# EUROPEAN PATENT SPECIFICATION

(11) **EP 3 027 234 B1**
(45) Date of publication and mention of the grant of the patent: **15.11.2017**
(21) Application number: 14749957.8
(22) Date of filing: 29.07.2014
(51) Int. Cl.: A61L 27/18, A61L 27/10, A61L 27/28, A61L 27/38, A61L 27/50, A61L 27/54, C12N 5/0775, A61C 8/00, A61L 31/08, A61L 31/14, A61K 31/695, C12N 5/077, A61K 31/131

(54) **OSTEOINDUCTIVE MATERIALS**
OSTEOINDUKTIVE WERKSTOFFE
MATÉRIAUX OSTÉO-INDUCTIFS

(30) Priority: 29.07.2013 GB 201313495
(43) Date of publication of application: 08.06.2016
(73) Proprietor: The University of Liverpool, Liverpool, L69 7ZX (GB)
(72) Inventor: CURRAN, Judith, Liverpool L69 3GA (GB); HUNT, John, Liverpool L69 3GA (GB)
(74) Representative: HGF Limited
(86) International application number: PCT/GB2014/052322
(87) International publication number: WO 2015/015189

(56) References cited:
- EP-A1- 2 609 941
- WO-A2-2007/048115
- WO-A2-2009/073068

## Description

The present invention relates to osteoinductive materials, and to methods for the production of such osteoinductive materials. The invention also relates to the use of the disclosed osteoinductive materials for the production of bone, and to methods of producing bone or bone precursor using such osteoinductive materials. A further aspect of the invention relates to medical uses of the osteoinductive materials.
Traditional approaches to the differentiation of cells *ex vivo* have tended to use naturally occurring biologically active substances such as growth factors to direct the differentiation of cells. Such growth factors, recombinant forms of which may typically be used in cell culture applications, tend to be costly. They are also frequently subject to degradation over time, which leads to them having short shelf lives.
There is an increasing recognition of the importance of the way in which materials can influence the activities of biological cells that are in contact with them, and an aim to develop materials that can influence these activities in a way that produces a desired effect.
For example it has been found that materials functionalised by the presence of -NH₂ groups attached to short hydrocarbon chains (three carbon chain length) are able to cause changes in mesenchymal stem cell gene expression profiles that are indicative of osteogenic differentiation. EP 2609941 aims at providing an osteoinductive material by grafting a layer of an aminoalkylsilane compound via O-Si-O bond onto the surface of the substrate. This document discloses a biocompatible implant for orthopaedic or dental use comprising titanium, which is covalently modified by an aminosilane compound. The aminosilane can be aminopropyltriethoxysilane.

Accordingly, there is growing interest in the use of biologically active materials in cell culture applications to direct the differentiation of cells in a manner that allows *ex vivo* generation of desired cell types or tissues.
Interest in such biologically active materials is not only limited to *ex vivo* applications. Medical implants represent a highly suitable context in which biologically active materials may be used to influence the activity of a patient's cells around the site of the implant.
The need for medical implants, such as orthopaedic implants, is expected to grow for the foreseeable future, as the aged population grows and expects to retain a high degree of mobility and quality of life. Some estimates suggest that the numbers of primary total hip and knee arthroplasty may grow respectively by 174% to 572,000 and by 673% to 3,480,000 procedures between 2005 and 2030 in the US.

Despite high clinical success rates associated with the introduction of orthopaedic implants, problems can arise that ultimately compromise the success of the implant in situ. Among these, one of the most common failings is insufficient integration between the material of the implant and the patient's tissue. This can cause loosening of the implant which may require corrective surgery or removal of the implant in order to be addressed.
Titanium is commonly used in the manufacture of implants, and exhibits excellent properties as an orthopaedic biomaterial. However, titanium implants are not osteogenic or osteoinductive. In an attempt to address this failing, numerous studies have investigated the modification of the titanium surface in order to enhance the implants bioactivity and hence optimise the bone-to-implant contact to provide complete integration and intimate bone apposition. Modification strategies reported in the prior art include blasting the alkaline surface with bioactive media, acid or alkaline etching, anodisation, and surface immobilisation of specific biofunctional molecules.
A common commercialised technique used for clinical orthopaedic and dental applications is plasma spraying of a (bioactive) calcium phosphate based coating, such as hydroxyapatite, onto the titanium surface to mimic the inorganic components of bone tissue. These techniques have achieved a certain level of success, and the surface-modified implants have been used clinically. However, the speed of bony ingrowth can be slow, delaying the patient's ability to fully utilise the implant.
Hydroxyapatite currently represents the "gold standard" for coating orthopaedic devices, and there is much research and development focused around methods of deposition of the hydroxyapatite coating, as well as suitable variations of hydroxyapatite coatings.
In view of the above it can be seen that there remains a need for new osteoinductive materials, The present invention relates to osteoinductive materials, and to methods for the production of such osteoinductive materials. The invention also relates to the use of the disclosed osteoinductive materials for the production of bone, and to methods of producing bone or bone precursor using such osteoinductive materials. A further aspect of the invention relates to medical uses of the osteoinductive materials.

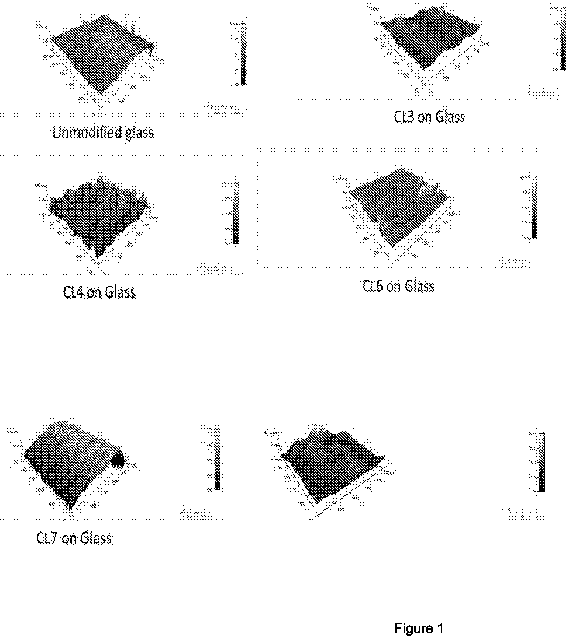

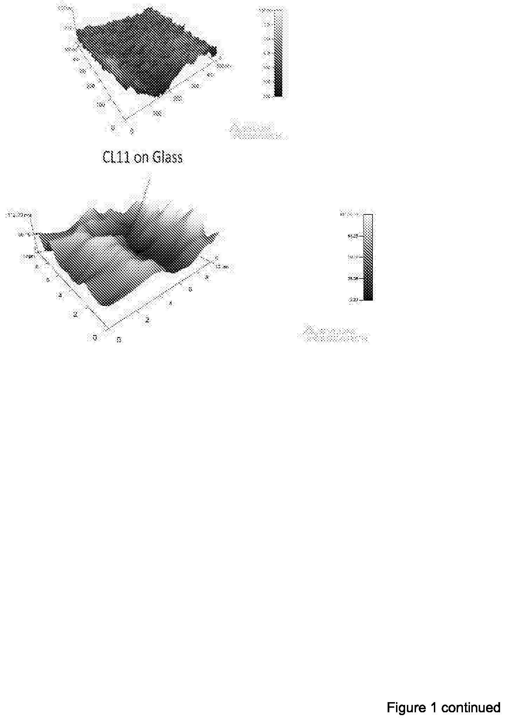

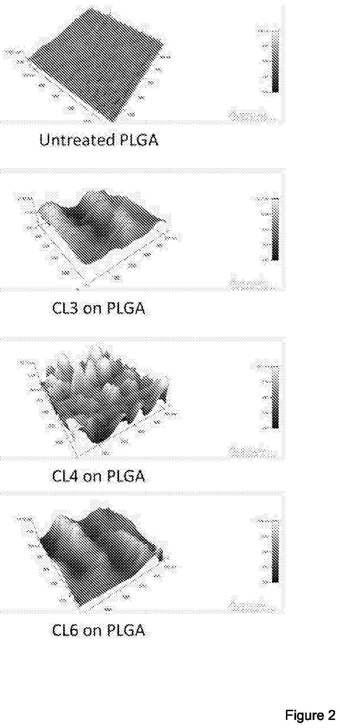

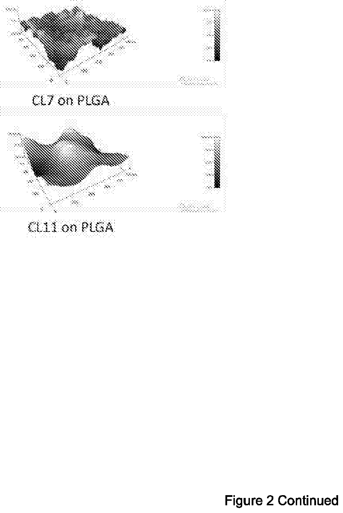

In a first aspect the present invention provides an osteoinductive material according to claim 1. An "osteoinductive" material in accordance with the present invention is one that induces cells with osteogenic potential to develop into the bone-forming lineage. In suitable embodiments the cells with osteogenic potential may be stem cells. Examples of suitable stem cells are described elsewhere in this application, and include mesenchymal stem cells. Suitable hydrocarbyl groups for use in the osteoinductive materials of the invention may be straight or branched. In a preferred embodiment the hydrocarbyl group is straight.
In a suitable embodiment the hydrocarbyl group is a 10 to 12 carbon alkyl chain, and in a particularly suitable embodiment that hydrocarbyl group is an 11 carbon alkyl chain.
In a suitable embodiment, the hydrocarbyl group referred to in connection with the various aspects of the invention is substantially the only hydrocarbon present on a coated surface of an osteoinductive material of the invention. In a suitable embodiment, the hydrocarbyl group is substantially the only hydrocarbon present on an osteoinductive material of the invention. In a suitable embodiment the hydrocarbyl group is attached to the substrate via a Si-O-Si bond.
The substrate employed in an osteoinductive material of the invention may comprise one or more constituents selected from the group consisting of: glass, metals; polymers; ceramics; and plastics. Suitable substrates may be determined with reference to the application in which the osteoinductive material is to be used, as discussed further below.
For the purpose of the present disclosure, except for where the context requires otherwise references to "metals" shall be taken as encompassing metal alloys. Thus, by way of example, osteoinductive materials according to the invention may employ a substrate comprising a metal selected from the group consisting of: steel; titanium; titanium alloys; and cobalt chrome.
The materials of the invention preferably comprise multiple layers of the hydrocarbyl group and presenting amine. These multiple layers tend to be distributed unevenly over the surface of the material, so that different depths of the layers occur over different regions of the material's surface. As a result, the multiple layers give rise to a number of nanometre-scale features on the surface of the osteoinductive materials of the invention. These features can be seen in the Figures described elsewhere in the specification (in particular in Figures 1 and 2) and have a height range of between approximately 50 and 155 nm (in particular materials of the invention utilising a polymer substrate may be characterised by the presence of nanometre-scale features having a height of between 50 nm and 155 nm; while materials of the invention utilising a glass substrate may be characterised by the presence of nanometre-scale features having a height of between 115 nm and 155 nm). This contrasts with the maximum height of nanometre-scale features found in comparison materials (produced using hydrocarbyl groups having chain lengths outside those specified in respect of the materials of the invention) which demonstrate a maximum height in the region of 35 nm.

Atomic force microscopy images of the surfaces of the materials of the invention indicate that the nanometre-scale features overlie a micrometer-scale topography.

The arrangement of the features on the surface of the material may also prove to be characteristic of the osteogenic materials of the invention. The features have an "open honeycomb" arrangement, as can be seen from microscopic images of the materials. This arrangement is also observed in the extracellular matrix components that biological cells deposit when cultured on the materials of the invention.

Without wishing to be bound by any hypothesis, the inventors believe that this "topography" of the surfaces of the osteoinductive materials of the invention, optionally in combination with features of the surface energy and surface chemistry of the materials described elsewhere in the specification, controls adsorption of proteins to the surface, which makes an important contribution to the biological activities exerted by the osteoinductive materials of the invention. This adsorption of proteins may be selective, in that it appears to particularly favour the adsorption of certain proteins, including (but not limited to) proteins selected from the group consisting of: fibronectin; and vitronectin.

In a suitable embodiment, an osteoinductive material of the invention has a maximum "feature height" at of least 40 nm.

In an alternative embodiment, an osteoinductive material of the invention has a maximum feature height of at least 60 nm.

In a still further embodiment, an osteoinductive material of the invention has a maximum feature height of at least 90 nm.

In a suitable embodiment, an osteoinductive material of the invention has a maximum feature height is between approximately 50 and 155 nm. This corresponds to a depth of the hydrocarbyl group on the surface of the osteoinductive material that ranges between approximately 50 and 155 nm.

In a suitable embodiment, an osteoinductive material of the invention has a maximum feature height is between approximately 90 and 150 nm. This corresponds to a depth of the hydrocarbyl group on the surface of the osteoinductive material that ranges between approximately 90 and 150 nm.

In suitable embodiments of the materials of the invention in which the substrate comprises a polymer, the maximum feature height may be approximately 100 nm. This corresponds to a maximum depth of approximately 100 nm of the hydrocarbyl group on the surface of the osteoinductive material.

Alternatively, embodiments of materials of the invention in which the substrate comprises a polymer may have maximum feature height of approximately 140 nm. This corresponds to a maximum depth of approximately 140 nm of the hydrocarbyl group on the surface of the osteoinductive material.

As referred to above, the inventors believe that the surface energies of the osteoinductive materials of the invention have an important impact (optionally in combination with the topography the materials' surfaces) upon the effects that they are able to exert upon biological cells. In a suitable embodiment an osteoinductive material of the invention may have a water contact angle at the surface of the material is less than 100°.

Suitably, an osteoinductive material of the invention may have a water contact angle at the surface of the material of between approximately 85° and 95°. In a particularly suitable embodiment the water contact angle at the surface of the material is approximately 90°.

These considerations regarding surface energy and water contact angles may be of particular relevance to embodiments of the osteogenic materials of the invention in which the substrate comprises a polymer.

In a second aspect, the invention provides a method of producing an osteoinductive material, the method comprising contacting a surface of a substrate with a solution containing a compound comprising a hydrocarbyl group comprising 10 to 12 carbon atoms and a terminal amine group, such that a coating of the hydrocarbyl group is formed on at least a portion of a surface of the substrate.

Suitable hydrocarbyl groups for use in these methods of the invention may be straight or branched. In a preferred embodiment the hydrocarbyl group used in the method is straight.

The hydrocarbyl group may be a 10 to 12 carbon alkyl chain, and in a preferred embodiment that hydrocarbyl group is an 11 carbon alkyl chain. In a particularly preferred embodiment the hydrocarbyl group is provided in the form of 11-aminoundecyltriethoxysilane.

In a suitable embodiment the method of the invention may be one, wherein the solution contains the hydrocarbyl group-containing compound (such as 11-aminoundecyltriethoxysilane) at a concentration of approximately 0.1 M. The inventors have found that methods in accordance with this embodiment give rise to osteodinductive materials that induce a response in cells that is homogenous across the surface that has been contacted with the hydrocarbyl group. It will be appreciated that this homogeneity of response confers advantages when the osteoinductive material is used, and so such homogenous materials will generally be viewed as being of increased quality. Without wishing to be bound by any hypothesis, the inventors believe that this property may be associated with a homogenous distribution of amine groups across the coated surface of the material. The distribution of amine groups can be determined using the ninhydrin assay as described elsewhere in the specification.

The methods of the invention may make use of any of the substrates considered elsewhere in the specification, including glass; metals; polymers; ceramics; and plastics.

Accordingly, the substrate may comprise a metal selected from the group consisting of: steel; titanium; titanium alloys; and cobalt chrome.

In a suitable embodiment the substrate may be in the form of an item that it is wished to provide with osteoinductive properties. For example, the substrate may be provided in the form of a medical implant (or part thereof) or a cell culture vessel (or part thereof).

A suitable manner in which the surface of the substrate may be contacted with the solution containing the hydrocarbyl group is by immersion of the substrate within the solution. In an alternative embodiment, the surface of the substrate may contacted with the solution by spraying the solution onto the surface of the substrate

Once the coating of the hydrocarbyl group has been formed, the method may further comprise washing the coated substrate in alcohol and/or water. The method of producing an osteoinductive material may further comprise drying the surface of the substrate.

For the purposes of the present disclosure, either an osteoinductive material in accordance with the first aspect of the invention, or an osteoinductive material produced by the methods of the second aspect of the invention, may be referred to as "an osteodinductive material of the invention". The osteoinductive materials produced by the methods of the second aspect of the invention may have any of the features described with reference to the first aspect of the invention.

In a suitable embodiment, an osteoinductive material of the present invention may be provided in the form of a medical implant, or a part thereof, such as those provided to a patient during surgery. Suitably an osteoinductive material of the invention in accordance with this embodiment may be in the form of an orthopaedic implant (or a part thereof). Alternatively an osteoinductive material in accordance with this embodiment of the invention may be in the form of a dental implant (or a part thereof).

When osteoinductive materials of the invention in the form of a medical implant are provided to a patient, they are suitable for use for the production of bone *in vivo* in accordance with the second aspect of the invention.

When an osteoinductive material of the invention is provided in the form of a medical implant, such as those described above, the hydrocarbon layer is preferably provided over some or all of the surface on which it is desired that bone should be produced. Merely by way of example, this may be a surface that is in contact with the patient's existing bone, in which case the production of new bone promoted by the osteogenic material of the invention may serve to integrate the implant with the existing bone, thus securing the implant.

An osteodinductive material of the invention may be provided in the form of a cell culture vessel, or a part thereof. Various forms of cell culture vessel are known to those skilled the art, including (but not limited to): cell culture dishes (such as Petri dishes); cell culture flasks; cell culture plates; cell culture bottles (such as roller bottles); cell culture bags; and cell culture beads.

Cell culture vessels in accordance with this embodiment may utilise as substrates any suitable material that is conventional in the manufacture of such vessels. Typically the material from which a cell culture vessel is manufactured will be a non-toxic, non-pyrogenic material. A suitable substrate for use in such cell culture materials may be selected from the group consisting of: glass; polystyrene; poly(lactic-co-glycolic acid) PLGA; poly(ether)urethanes; polyesters; and polycaprolactone.

When an osteoinductive material of the invention is provided in the form of a cell culture vessel, such as those described above, the hydrocarbon layer is preferably provided over some or all of the surface on which cells are grown. Cell culture vessels comprising an osteoinductive material of the invention are particularly suitable for use in the uses of the invention, in which bone is produced *ex vivo,* such as *in vitro.*

In the event that the substrate is in the form of a part of a cell culture vessel, this part may be a portion that in use will come into contact with cells to be cultured.

In a third aspect, the invention provides the use of an osteoinductive material of the invention for the production of bone or a bone precursor. In the context of the present disclosure "a bone precursor" may be taken as encompassing the extracellular matrix that is found prior to ossification. A bone precursor of this sort may be characterised by the presence of extracellular osteocalcin, and/or by the calcification of the extracellular matrix. For the sake of brevity, references to "bone" in the present specification should also be taken as encompassing "bone precursor", except for where the context requires otherwise.

The production of bone in the context of the third aspect of the invention may be production of bone *in vivo,* or production of bone *ex vivo,* such as production of bone *in vitro.*

In a fourth aspect, the invention provides a method of producing bone or a bone precursor, the method comprising:
- providing an osteoinductive material according to the invention;
- providing a population of cells with osteogenic potential; and
- maintaining the cells in contact with the material until bone or a bone precursor is produced.

Generally, in the uses and methods of the third and fourth aspects of the invention, bone will be produced if the use or method in question is continued after bone precursor has been formed. Thus the methods or uses may further comprise maintaining cells in contact with the material until the bone precursor is remodelled into bone or a bone-like substance.

It will be appreciated that these methods of the invention provide many of the same advantages that have already been described in connection with the osteoinductive materials of the invention. In particular the methods of the invention increase the consistency and reproducibility of the bone produced, since the materials exert an inherent osteoinductive effect upon the cells, rather than requiring the addition of supplements (the potency of which can vary significantly between batches) to induce bone production. The fact that the methods of the invention make it possible to avoid the need for use of cell culture supplements, such as growth factors, also provide economic advantages in that supplements of this sort are frequently very expensive.

As described elsewhere in the specifications, the materials of the invention have the ability to absorb calcium and phosphate onto their surfaces when placed in an environment that contains these substances. This absorption contributes to formation, by cells in contact with the material, of a very dense extracellular matrix on the surface of the material. As illustrated further in the Experimental Results section (in particular the results of x-ray analysis and von Kossa staining, images of which show very early positive staining), this effect is only observed in connection with materials of the invention (exemplified by experimental materials comprising an eleven carbon hydrocarbyly group) and not in other control materials (such as materials comprising hydrocarbyl groups with shorter carbon chain lengths).

The biological cells deposit and become encased in this extracellular matrix, and start to remodel the matrix, forming an even denser osteogenic matrix. It will be appreciated that the ability of the materials of the invention to stimulate the production of calcium/phosphate rich matrices that are highly osteoinductive has many useful applications. The methods by which these materials of the invention may be produced, using simple wet chemistry and adsorption, are significantly cheaper than other approaches using expensive hyaluronic acid (HA)/calcium phosphate (CaP) coating technologies.

In a suitable embodiment, the cells with osteogenic potential are stem cells. Merely by way of example, such stem cells may be selected from the group consisting of: embryonic stem cells; cord blood stem cells; adult stem cells (including haematopoietic stem cells; mesenchymal stem cells; and dental pulp stem cells); and induced pluripotent stem cells.

In a preferred embodiment of the methods of the invention, the stem cells are mesenchymal stem cells. Mesenchymal stem cells represent particularly suitable cells that may be induced to produce bone by the appropriate methods or uses of the invention, in that they have been shown to undergo strong osseoinduction in response to contact with the materials of the invention. Furthermore, such mesenchymal stem cells tend to be quite abundant at the sites where medical implants are inserted, since they are found at high numbers in the bone marrow and dental pulp. Furthermore, mesenchymal stem cells are considered very favourably as cells for use in *ex vivo* applications, since they can be obtained in relatively high numbers from sources that do not tend to cause ethical concerns (including sources such as cord blood or adipose tissue, as well as the bone marrow and dental sources mentioned above), are capable of expansion in culture, and have shown properties that make them suitable for transplantation.

In a suitable embodiment of the methods of the invention, the material of the invention and cells that are to be induced to form bone are provided with a source of phosphate and/or a source of calcium. In embodiments where the production of bone is taking place *ex vivo,* a suitable source of phosphate and/or calcium may be provided in a cell culture medium.

Suitably the cells and osteoinductive material are maintained in serum-free cell culture medium. Many existing protocols for the maintenance, expansion or differentiation of stem cells require the use of serum as a supplement to ensure cell growth. However, serum contains many factors that can have unwanted impact upon cells to which it is provided. Furthermore, since serum is derived from animal sources it is not suitable for use in the context of culturing cells that will be provided therapeutically, since there is the risk that the serum may contain infective agents that will contaminate the cells.

The ability of the materials of the invention to promote osteogenesis in serum-free culture conditions is surprising in that use of serum is a pre-requisite of many prior art techniques, and is also highly advantageous in that it enables the cultured cells to be used therapeutically avoids the problems of unwanted or unknown influence on cell activity.

In a suitable embodiment, a method of the invention may be one in which the cells are maintained in cell culture medium without osteogenic growth factors. It will be appreciated that the osteoinductive properties of the materials of the invention may render further osteogenic factors, such as grown factors, redundant. Indeed, in a suitable embodiment, a method of the invention may be one wherein the cells are maintained in cell culture medium substantially free of exogenous growth factors. Such methods provide advantages in that they avoid the costs associated with the addition of growth factors, such as osteogenic growth factors and also the unwanted variability that may arise when different concentrations or potencies of growth factors are used.

In a fifth aspect, the invention provides a method of promoting integration of a medical implant, the method comprising providing to a subject in need of such treatment a medical implant comprising an osteoinductive material of the invention.

It will be appreciated that the osteoinductive materials of the invention are suitable for use as medical implants in contexts where it is desirable for integration of the medical implant to be promoted. The ability of the osteoinductive materials of the invention to promote bone growth will be expected to provide advantages in terms of increases in the speed and extent of osteointegration that occur on introduction of the medical implant into its site in the patient. These increases may be expected to improve the effectiveness and longevity of such implants.

The invention will now be further described with reference to the following experimental results section, and the accompanying Figures, in which:
Figure 1 shows atomic force microscopy (AFM) images of the surfaces of osteoinductive materials of the invention incorporating glass substrates. Changes in nanotopography associated with the different lengths of carbon chains presenting amine groups can clearly be observed. On chain lengths CL7 and CL11 maximum peak heights exceeded 10nm and larger scale images were taken to assess the effect on microtopography as well as associated nanotopography. Images clearly show that chain lengths 7 and 11 resulted in a different surface nanotopography on top of a micro topography.
Figure 2 shows atomic force microscopy (AFM) images of the surfaces of osteoinductive materials of the invention incorporating polymer (PLGA) substrates. Changes in nanotopography associated with the different lengths of carbon chains presenting amine groups can clearly be observed. Analysis of maximum peak heights is shown in Figure 7.
Figure 3 is a graph showing quantitative analysis of maximum peak heights is provided in Figure 1. Quantitative analysis of maximum height associated with different chain lengths was measured by AFM. Evaluation of the changes in height associated with the different chain lengths across entirety of the surface are shown in this Figure.
Figure 4 is a graph showing quantitative analysis of maximum peak heights is provided in Figure 2. Quantitative analysis of maximum height associated with different chain lengths was measured by AFM. Evaluation of the changes in height associated with the different chain lengths across entirety of the surface are shown in this Figure.
Figure 5 shows scanning electron microscopy (SEM) images of film structure on materials (specifically, a control, experimental materials, and an osteoinductive material of the invention) incorporating a glass substrate. Nanometre-scale changes to the film structures are beyond the sensitivity of this technique, indications of gross changes in film topography associated with CL11, changes are proven by AFM analysis in Figure 1.
Figure 6 shows scanning electron microscopy (SEM) images of film structure on materials (specifically, a control, experimental materials, and an osteoinductive material of the invention) incorporating a PLGA substrate. A change in film architecture can be obsereved with CL11 modifications (the osteoinductive material of the invention), a pattern that is repeated by matrix deposition associated with an enhanced osteogenic response induced by the material of the invention.
Figure 7 is a graph showing water contact angle of the different chain length amines on glass substrates. Each chain length results in a specific change in associated surface energy as measured by water contact angle.
Figure 8 is a graph showing water contact angle of the different chain length amines on a PLGA substrate. Amine modifications increase the hydrophilic nature of the PLGA film, each chain length results in a specific change of associated surface energy as measured by water contact angle. There is a specific surface energy, in the range 85-95, associated with the CL11 chain length.
Figure 9 is a graph comparing the results of X-ray analysis of phosphate absorption onto materials of the invention, and comparator materials, from various concentrations of phosphate buffered saline solution.
Figure 10 shows micrographs of von Kossa staining of hMSC cultured on experimental materials utilising glass substrates for 7 (panel A) and 28 (panel B) days.
Figure 11A-E fluorescent microscopy of hMSC on experimental materials utilising glass substrates at 14 or 28 days
Figure 12 shows micrographs of von Kossa staining of hMSC cultured on experimental materials utilising polymer (PLGA) substrates for 7 (panel A) and 28 (panel B) days.
Figure 13A-B fluorescent microscopy of hMSC on experimental materials utilising polymer substrates at 14 or 28 days
Figure 14 shows scanning electron microscopy images of osteoblasts cultured on a control material for 7 days.
Figure 15 shows scanning electron microscopy images of osteoblasts cultured on an experimental comparator material (CL3) for 7 days.
Figure 16 shows scanning electron microscopy images of osteoblasts cultured on an experimental comparator material (CL4) for 7 days.
Figure 17 shows scanning electron microscopy images of osteoblasts cultured on an experimental comparator material (CL6) for 7 days.
Figure 18 shows scanning electron microscopy images of osteoblasts cultured on an experimental comparator material (CL7) for 7 days.
Figure 19 shows scanning electron microscopy images of osteoblasts cultured on a material of the invention (CL11) for 7 days.
Figure 20 compares micrographs showing von Kossa staining of osteoblast populations cultured on control or experimental comparator materials, or a material of the invention, for 7 days.
Figure 21 is a graph comparing the formation of nodules among osteoblast populations cultured on control or experimental comparator materials, or a material of the invention, for 7, 14, or 28 days.
Figure 22 shows scanning electron microscope (SEM) images of the surfaces of control materials (labelled "unmodified" in A and B), comparator materials (labelled "3(Aminopropyl)triethoxysilane" in C and D), and materials of the invention (labelled "11-Aminoundecyltriethoxysilane") formed on titanium substrates at different magnifications (panels B, D, and F are at greater magnification than panels A, C, and E).
Figure 23 shows photomicrographs of MSCs cultured for 28 days on titanium substrates either untreated (control material labelled "Unmodified Ti"), or treated with silanes to yield comparator materials (labelled "3-(Aminopropyl)triethoxysilane") or osteoinductive materials of the invention (labelled "11-Aminoundecyltriethoxysilane").

### Experimental results

### Study I

### 1 Preparation of osteoinductive materials of the invention and experimental comparison materials

### 1.1 Preparation of materials on glass substrates

Glass was cleaned using a 0.5M solution of sodium hydroxide (Sigma, UK) for 30 minutes in an ultrasonic bath, the samples were then washed in three changes of distilled water, and placed in 1M Nitric acid for 30 minutes in an ultrasonic bath. Samples were then washed with three changes of distilled water and dried in a 50°C oven. Clean coverslips were then modified using the silanes in Table 1 in 0.1M solutions for 30 minutes.

The coverslips modified using 11-aminoundecyltriethoxysilane represent osteogenic materials of the invention (and the manner in which they have been manufactured represents a method of the invention), while the coverslips modified using other carbon chain lengths (CL3, CL4, CL6, and CL7) constitute experimental comparison materials.

Samples were then washed with Isopropyl alcohol for 5 minutes and then washed with distilled water prior to use.

### 1.2 PLGA film production and preparation of materials on PLGA

Clean 12mm glass cover slips were coated with chromium using Emtech 575x sputter coater (Emtech, UK), to provide a surface for the PLGA film to form hydrogen bonds with. 100 µl of 10 % 85:15 PLGA (Sigma, UK) in chloroform (UOL chemical stores, UK) was spin coated onto the chromium coated cover slips using WS-400B-6NPP/LITE spin coater (Laurell Technologies Corporation, UK). Oxygen plasma was used to functionalize the polymer (Emtech, UK), at the previously optimised settings of 30kW for 2 minutes.

As with the glass substrates described above, clean coverslips coated with PLGA were then modified using the silanes in Table 1 in 0.1M solutions for 30 minutes.

The coverslips modified using 11-aminoundecyltriethoxysilane represent osteogenic materials of the invention (and the manner in which they have been manufactured represents a method of the invention), while the coverslips modified using other carbon chain lengths (CL3, CL4, CL6, and CL7) constitute experimental comparison materials.

Samples were then washed with Isopropyl alcohol for 5 minutes and then washed with distilled water prior to use.

**Table 1**

| | |
|---|---|
| CL3 | (3-Aminopropyl)triethoxysilane (Sigma) |
| CL4 | 4-(triethoxyslyl(butan-1-amine (fluorochem) |
| CL6 | 3-(2-Aminoethylamino) Propyldimethoxymethylsilane (Sigma) |
| CL7 | N-(6Aminohexyl)amnomethyltriethoxysilane (fluorchem) |
| CL11 | 11-Aminoundecyltriethooxysilane (fluorochem) |

### 1.3 Von Kossa Stain

Samples were hydrated using 3 submersions in distilled water for 2 minutes each, then covered with 2% silver nitrate solution (Sigma UK) and placed under a UV lamp for 1 hour. Samples were washed with distilled water and put in a 2.5% sodium thiosulphate solution (Sigma UK) for 3 minutes. Samples were then washed with distilled water and counterstained using Harris's haematoxylin (Sigma UK) for 5 minutes, washed in running tap water for 5 minutes and differentiated in 1% acid alcohol for 2 seconds before being washed in distilled water. Samples were then dehydrated through increasing concentrations of alcohols (70%, 90% and 100%) for 2 minutes each and cleared in xylene (BDH, UK) for 2 minutes then mounted with glass coverslips in DPX (BDH, UK).

### 2 Analysis of physical and chemical properties of the experimental materials

### 2.1 Atomic Force Microscopy (AFM) and Measurements

Images obtained via atomic force microscopy of experimental materials formed on glass or polymer substrates are respectively shown in Figures 1 and 2. Of these experimental materials, those designated CL11 represent materials of the invention.
It can be seen that the distribution of the hydrocarbyl chains and presenting amine groups is not uniform, over the surface of the materials, giving rise to features having a "wave-like" appearance when visualised by AFM. The uneven distribution is observed in respect of the various experimental materials, but distinctions can be made between the features found on materials of the invention, and those found on comparator or control materials.

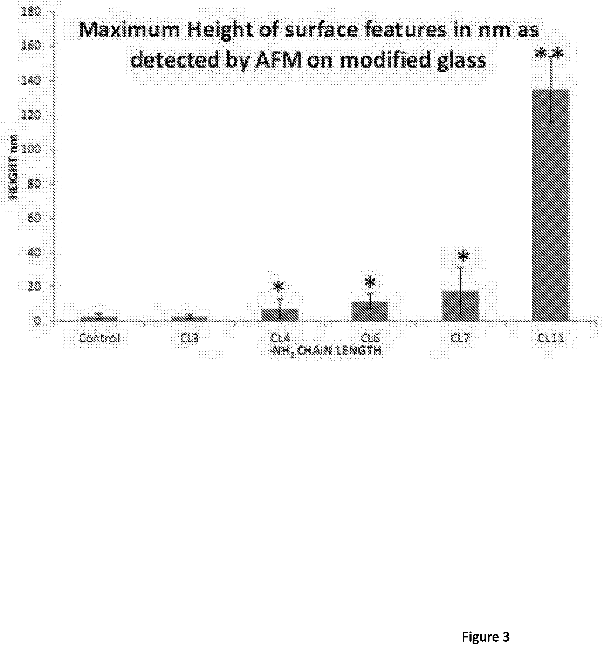

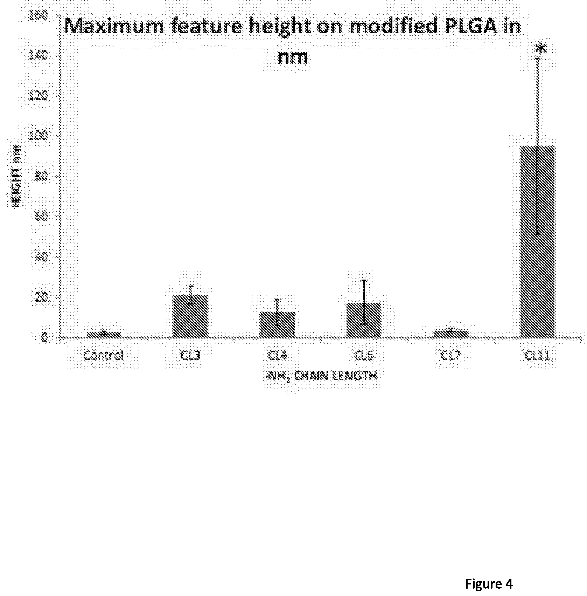

Quantification of the maximum heights of the features on the surfaces of the experimental materials (as measured by AFM) is shown in Figures 3 and 4. Here it can be seen that the maximum feature height in materials of the invention is significantly larger than the maximum height achieved in control or comparator materials.

### 2.2 Scanning Electron Microscopy

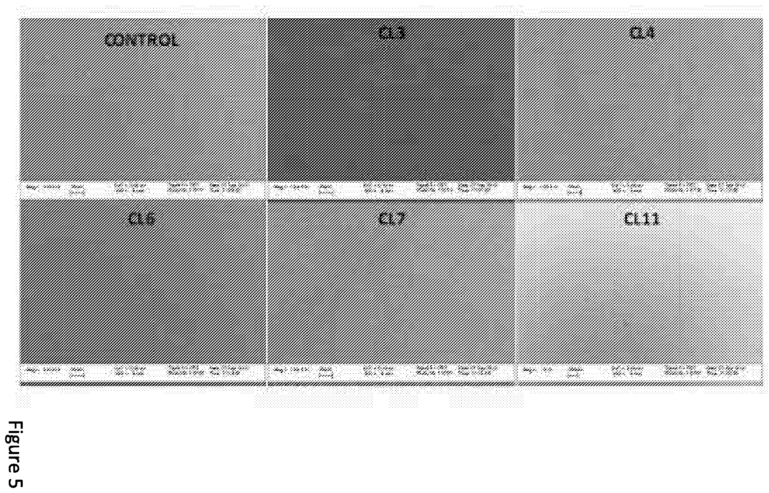

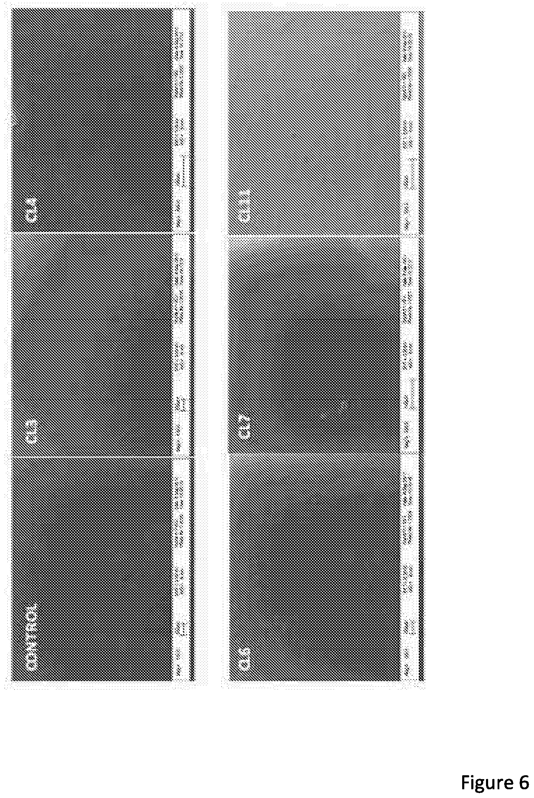

The surfaces of control and experimental materials (including a material of the invention designated CL11) were investigated using scanning electron microscopy. As can be seen from the representative images shown in Figures 5 and 6, the features on the surface of the materials were below the size that can be resolved by scanning electron microscopy, confirming that the features are on a micrometre and nanometre scale, as illustrated in the AFM images.

On materials of the invention (the CL11 modified substrates) there is clear evidence of a network formation on both the glass and PLGA modified substrates, representative of the open honeycomb structure described in later sections. This was more apparent on materials of the invention using a PLGA polymer substrate, but enhanced contrast of the images of the materials of the invention based on glass substrates clearly shows the presence of this network structure on the surface. This is not observed on any of the other modified or control substrates, therefore is a distinguishing feature of the materials of the invention.

### 2.3 Water Contact Angle (WCA) Measurements

Double sided materials were used for this technique, produced in the same way as previously described. WCA were measured using a Camtel Dynamic Contact Angle (DCA) machine (Camtel LTD, UK). 6 replicates were carried out per repeat, a total of 4 repeats were carried out for each test substrate. Values provided are average +/- standard deviation. Statistical analysis was carried out using ANOVA Waller Duncan and Tukey models.

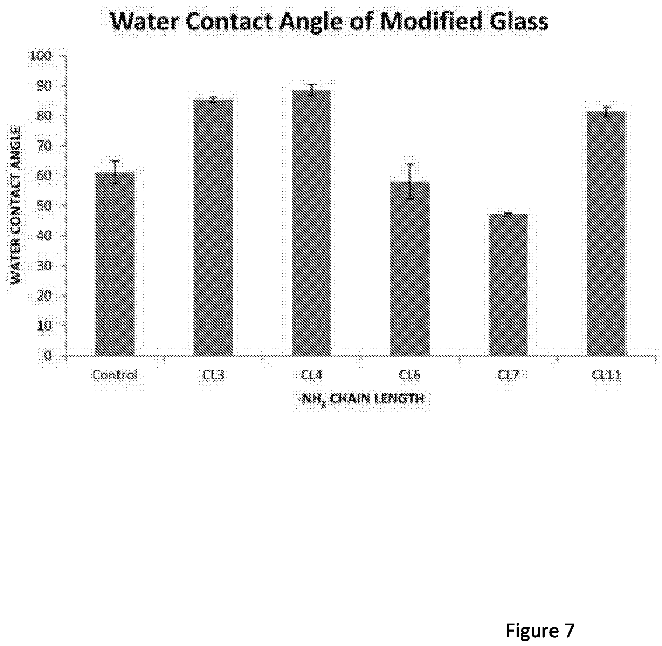

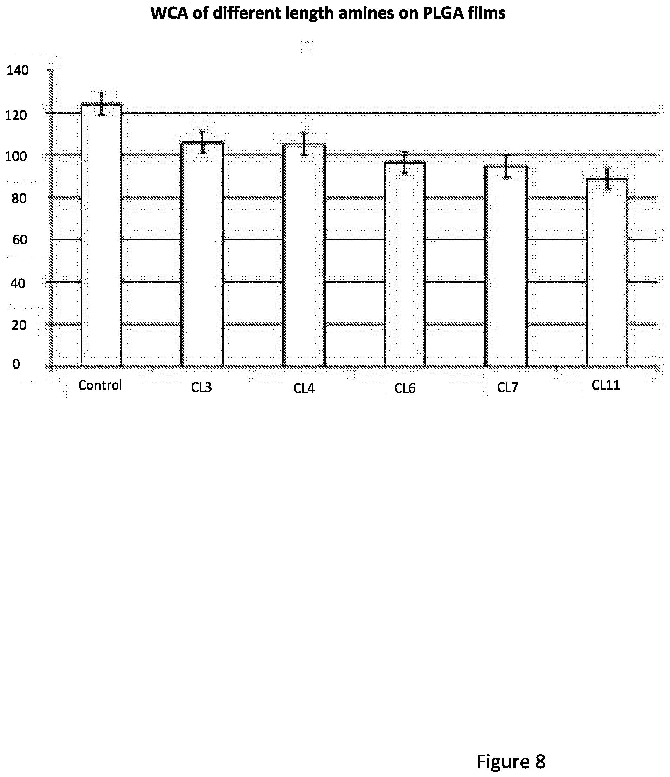

The results obtained with experimental materials using glass or polymer substrates are respectively set out in Figures 7 and 8. Here it can be observed that particularly in the results obtained from polymer-based materials (Figure 8) the surface energy of the materials of the invention (CL11) is distinctly lower than the surface energy of other comparator materials.

### 2.4 X-ray Analysis of Glass (Control) and Modified Films

Dry modified films and glass coverslips were coated with carbon using a carbon coater (EMTECH, UK). X-ray microanalysis was performed using a Leo 1550 SEM (Zeiss, UK) with an INCA system (Oxford Instruments). Points of analysis were imaged at 5 locations on the film. From each of these fields 10 spectrums were generated at random. The resulting data was analysed using ANOVA, to determine any statistically significant difference between the elements on the scaffolds.

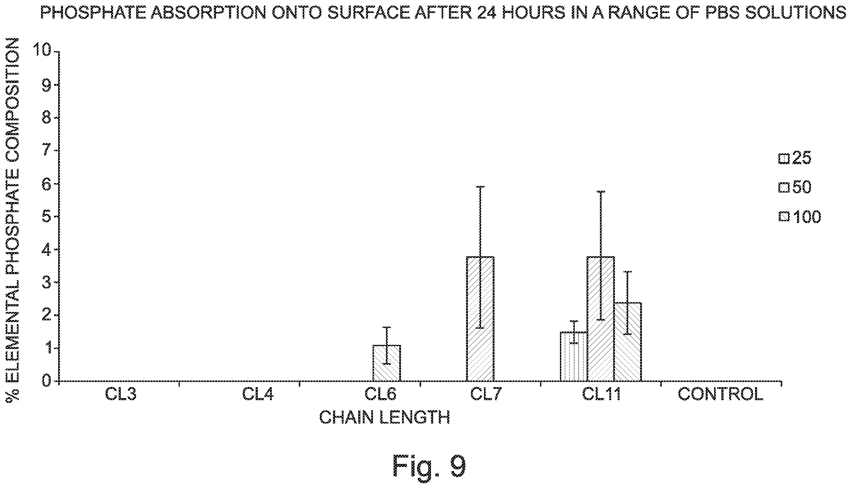

Phosphate deposition is relevant to its role in the formation of an osteoinductive matrix that favours bone formation. As can be seen from Figure 9, phosphate absorption onto the surfaces of experimental materials was only observed in respect of the materials of the invention (CL11), and comparator materials incorporating six and seven carbon chains. However, while phosphate absorption was observed in respect of each of these materials, the extent of phosphate absorption, and range of conditions (particularly with respect to surrounding phosphate concentration) in which phosphate absorption occurs, was greater for the materials of the invention than for comparators.

### 3 Analysis of biological properties of the experimental materials

### 3.1 Mesenchymal Stem Cell Response

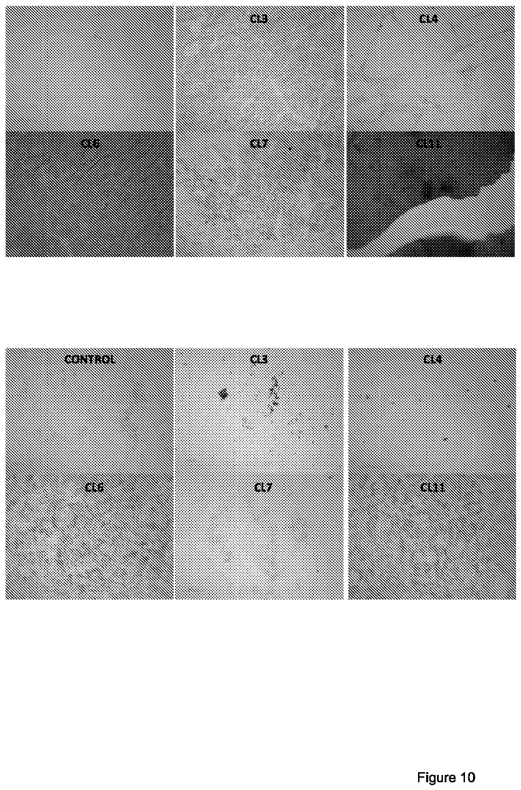

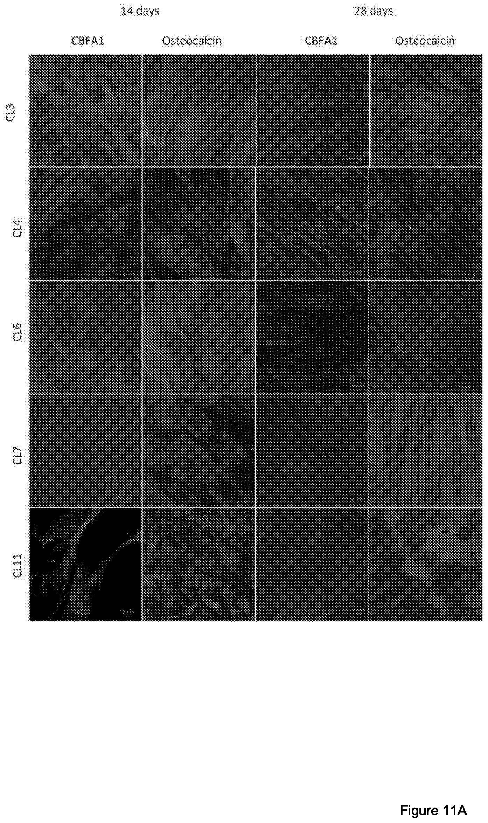

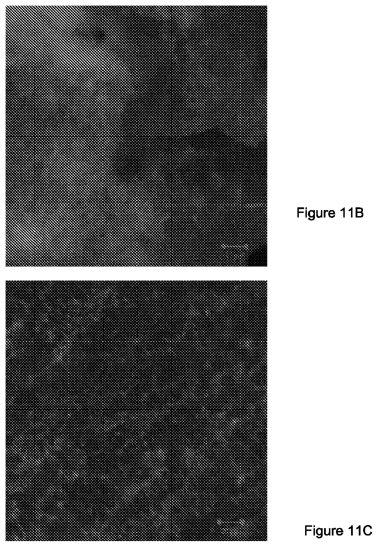

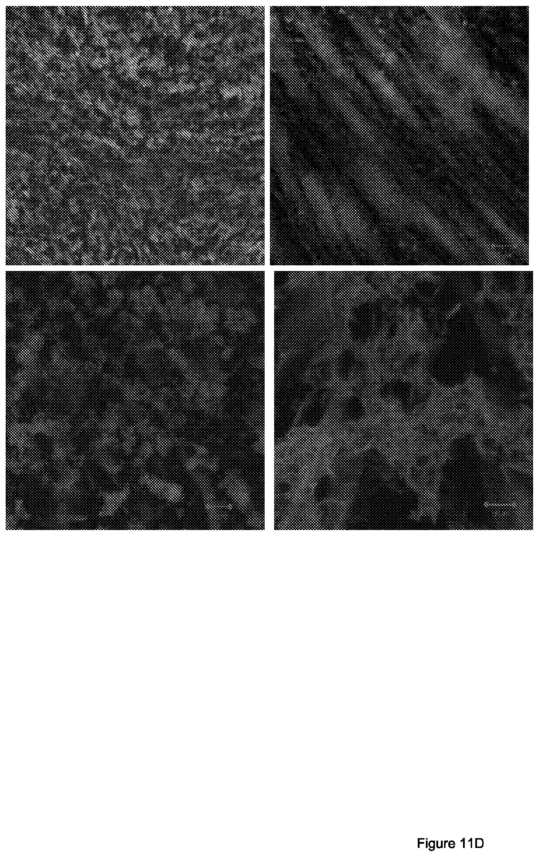

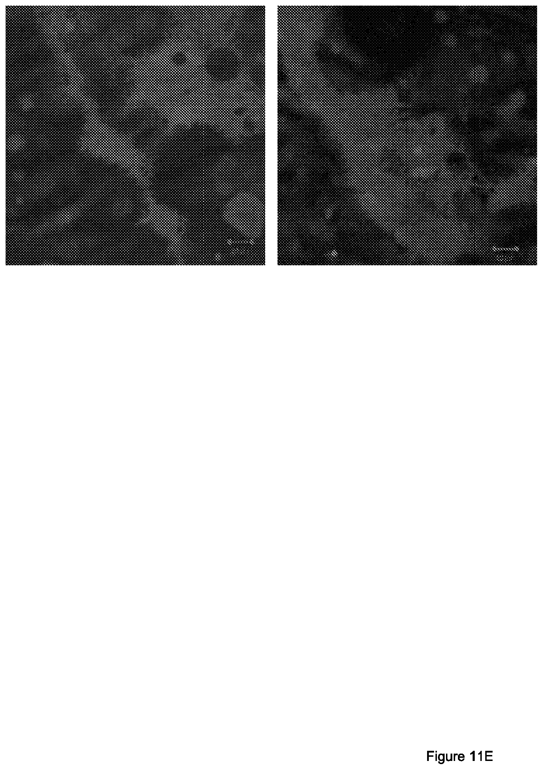

Mesenchymal stem cells were cultured on experimental materials (utilising glass or polymer substrates) for 28 days. The experimental materials included materials of the invention (CL11). Cells were investigated at various timepoints, and the results are shown in Figures 10 and 11.

Panels A and B of Figure 10 respectively show von Kossa staining of human mesenchymal stem cells (hMSCs) cultured for 7 and 28 days on the various experimental and control materials (CL11 representing a material of the invention).

Positive von Kossa staining (indicating the presence of calcified extracellular matrix) is indicated by a dark brown/black colour. It can be seen in Figure 10A that, after 7 days in culture, a denser matrix is observed on the CL11 sample than is found on the other control or comparator materials.

Turning now to Figure 10B, von Kossa positive calcified extracellular matrix is once again stained with a dark brown/black colour. There is clear evidence of remodelling and reorganisation of the calcified extracellular matrix on the CL11 material (of the invention), indicating the osteoinductive properties of this material. In contrast, while the comparator material made with a six carbon chain also shows evidence of a calcified extracellular matrix, other indications suggest that this is not associated with efficient osteoinduction or formation of bone-like material.

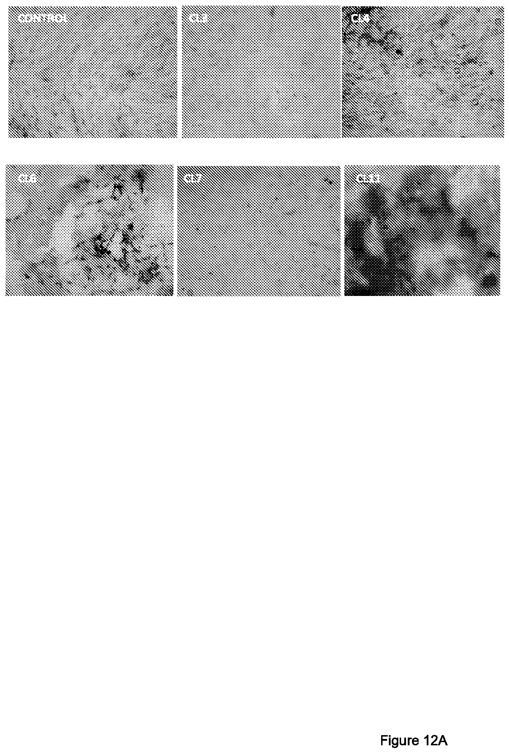

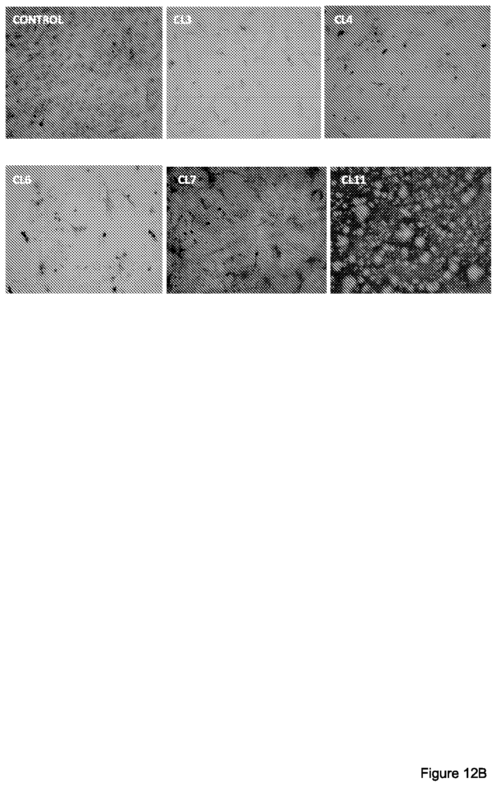

Materials of the invention produced on a polymer substrate are able to induce a similar response in cultured hMSCs, as shown in panels A and B of Figure 12. Indeed, the distinction between the effects observed in cultures grown on materials of the invention, and those grown on comparator or control materials, are even more pronounced in this case.

While cells adhere to and grow on the other materials (as shown by haematoxylin and eosin staining), deposition of calcified extracellular matrix is only observed in respect of the material of the invention (CL11). Here the pattern of the distribution of the matrix reflects the underlying topography of the features found exclusively in the material of the invention.

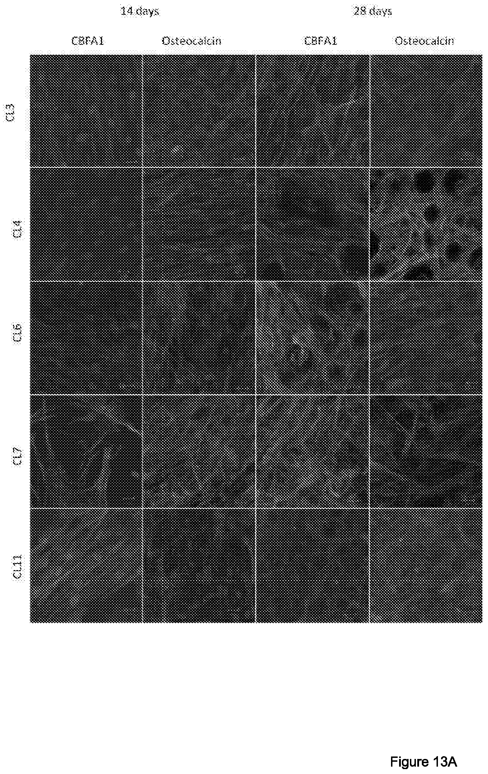

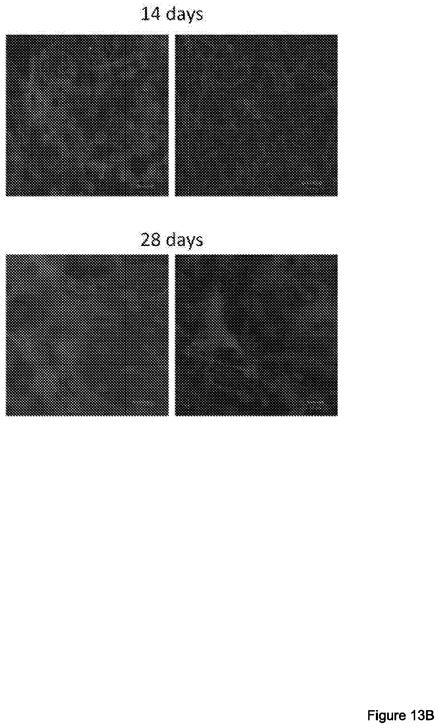

Figures 11 and 13 illustrate immunofluorescent labelling of hMSCs grown on a range of experimental materials (including a material of the invention designated CL11) utilising either glass (Figure 11) or PLGA polymer (Figure 13) substrates. In each case, images are shown for cells cultured for either 14 or 28 days on the materials. Cell nuclei are stained with DAPI (blue colour), and cells have also been labelled for the presence of the transcription factor CBFA1 (red colour in cell nuclei), cytoskeletal actin (green colour), and ostocalcin a major component of the osteogenic extracellular matrix (red colour).

The images set out in Figures 11 and 13 clearly show that only cells grown in contact with the materials of the invention (whether on glass or polymer substrates) form an osteocalcin rich matrix when cultured *in vitro* under basal conditions. While osteocalcin staining is found in cells grown on other materials, this has an intracellular location and so cannot be associated with the formation of an osteocalcin-rich extracellular matrix (which is a definitive marker of osteoinduction).

In contrast, the osteocalcin produced by cells grown on materials of the invention is deposited in a dense extracellular matrix. Even by day 7, the cells can be seen to be embedded within this matrix (see Figure 11B), and at the cells subsequently reorganise and remodel this matrix. By 28 days this leads to the formation of cell-containing pits within the surface of the matrix, indicative of the cells undergoing a change from an osteoblast to osteocyte phenotype. Osteocalcin arrangements illustrating these changes can be seen in images taken after 14 or 28 days in culture (right hand columns of Figures 11A and 13A, Figures 11B-11E and 13B).

### 3.2 Human Osteoblast Response

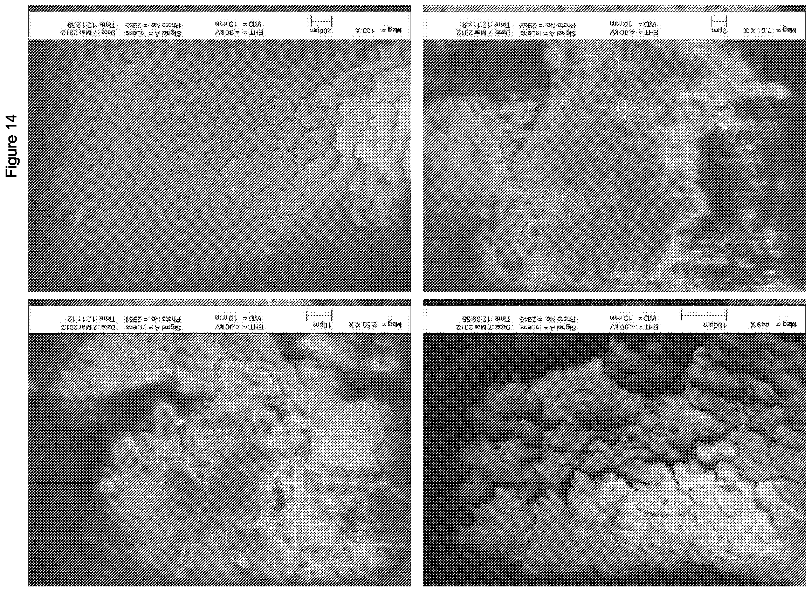

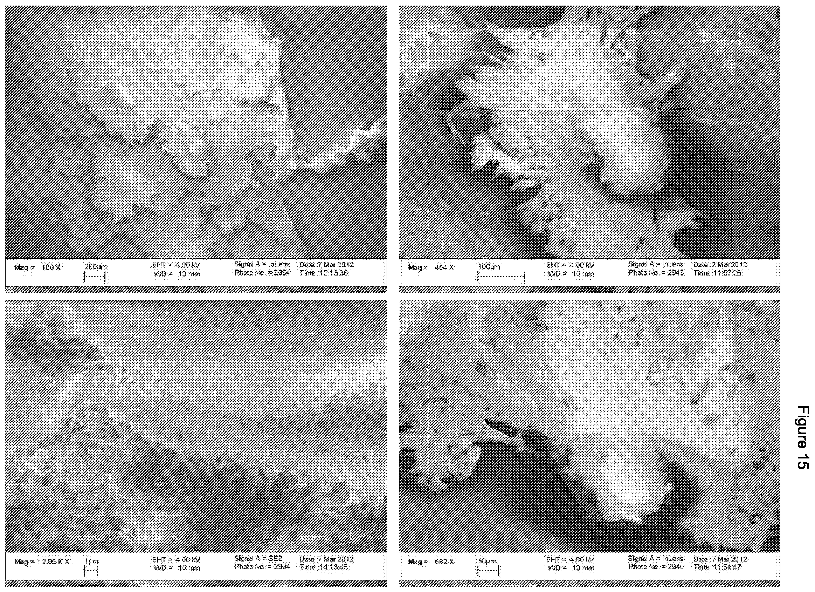

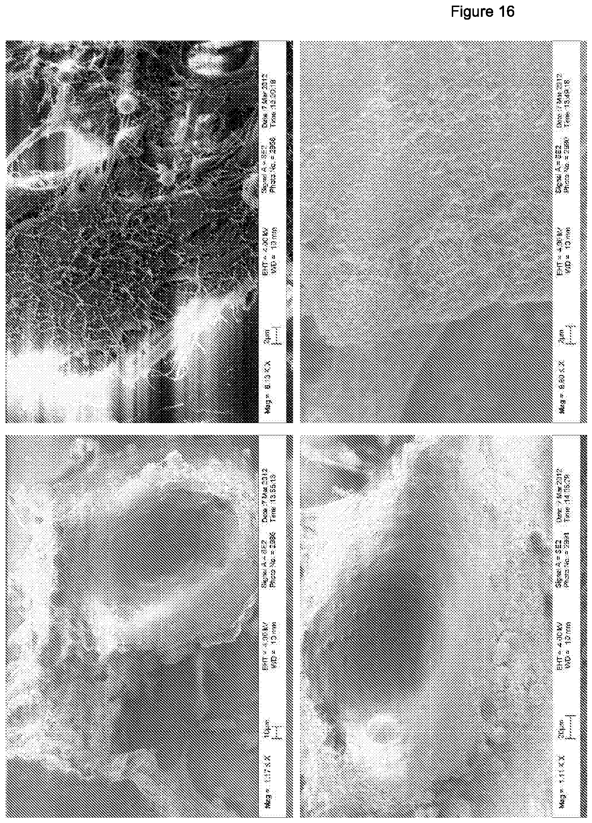

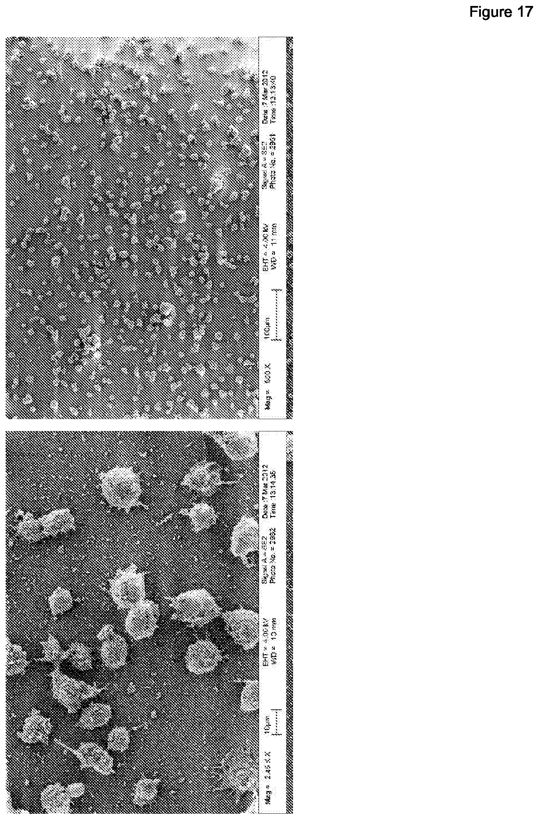

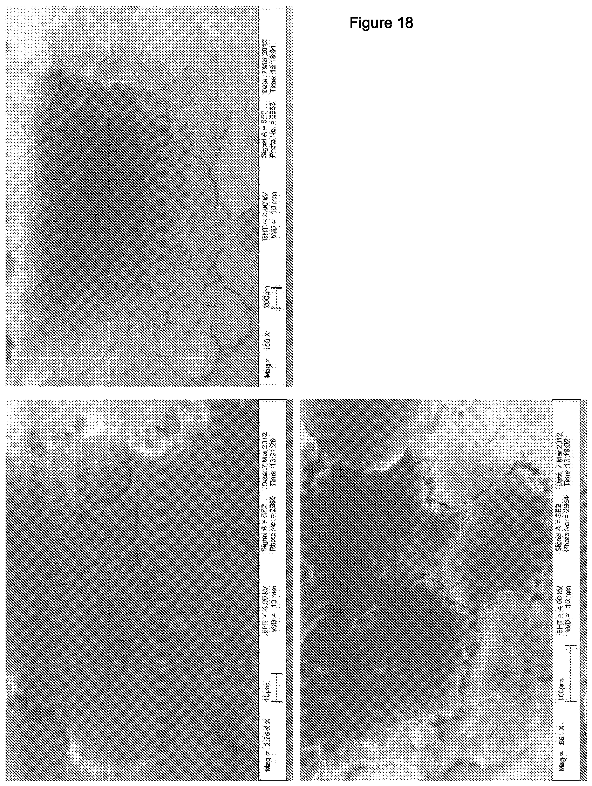

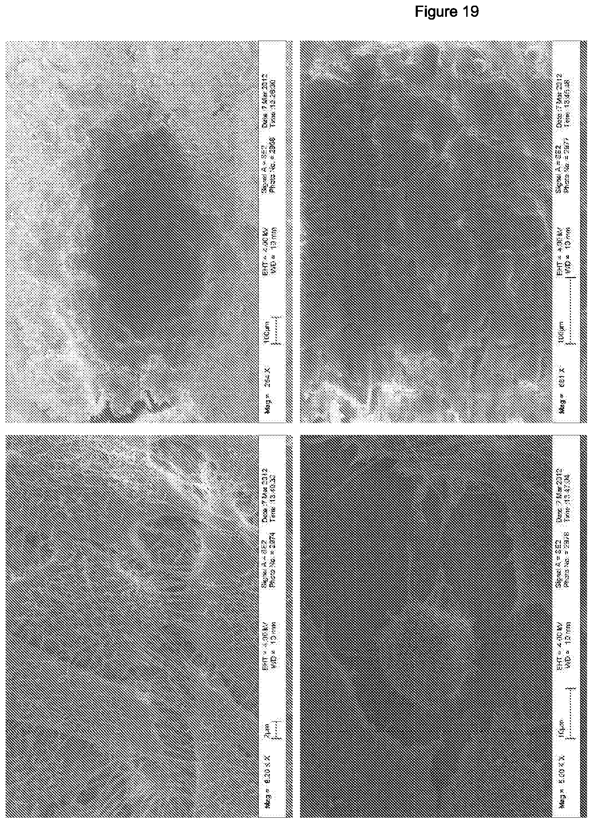

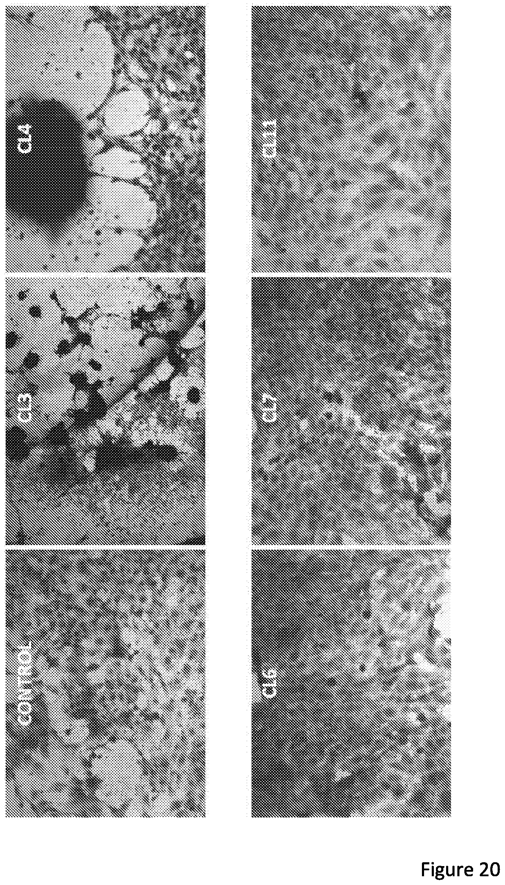

Primary derived human osteoblasts were also cultured on experimental materials (including a material of the invention designated CL11) for various periods of time (up to 28 days). Scanning electron micrographs illustrating representative populations of these cultured cells after 7 days are set out in Figures 14 to 19, and von Kossa staining of 7 day cultures is shown in Figure 20.

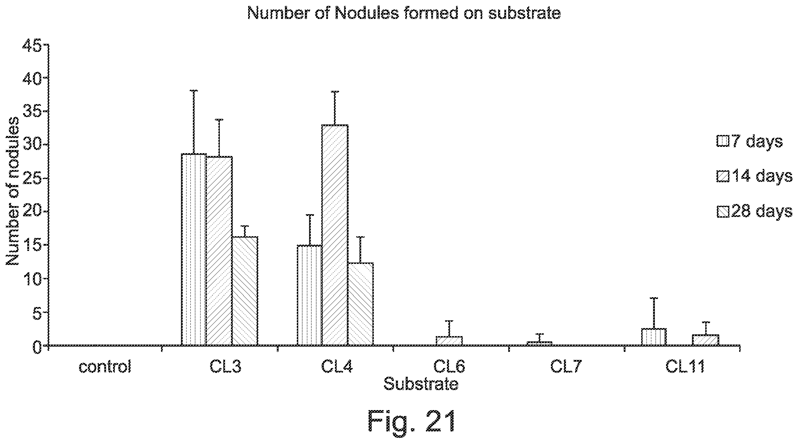

When cells were cultured in contact with CL11 materials (osteoinductive materials in accordance with the invention) there was no observable difference in the cellular response when compared to control, CL6 and CL7 substrates. All of these substrates supported osteoblast attachment and growth but did not result in the formation of calcified cell clusters as observed on CL3 and CL4 substrates. Therefore the observed osteoinductive effect associated with materials of the invention when used as a surface for stem cell growth did not result in the significant enhancement of osteoblast cell culture. This phenomenon i.e. enhanced calcified matrix and nodule production by osteoblasts was only associated with the specific material parameters produced by CL3 and CL4 modifications. Nodule formation was quantified using SEM and image analysis and the results are set out in Figure 21.

### Study II

### 4 Preparation of further controls, comparators, and osteoinductive materials of the invention

The data presented in Study I (above) illustrate that optimal -NH₂ surface modification parameters (silane modification of a base substrate with 11-Aminoundecyltriethoxysilane) enable to production of materials of the invention with enhanced osteoinductive properties on glass or tissue culture polystyrene (TCPS) substrates. The current Study confirms that the process of modification is translatable to metallic, titanium, substrates, resulting in the generation of osteoinductive materials of the invention that offer superior properties to comparator materials made using other -NH₂ modifications (employing hydrocarbyl groups with a length other than 10 to 12 carbon atoms) on the same substrates.

### 4.1 Methods

### 4.1.1 Material Modification

5.0mm diameter polished titanium (Ti) discs were immersed into a 10% NaOH solution for 1 hour then washed thoroughly with high pure water and dried with nitrogen, then stored in a vacuum desiccator prior to introduction of amine groups (NH₂) on the surface by silanation. To introduce the -NH₂ Ti discs were dipped into 2% silane 1 (3-Aminopropyl)triethoxysilane) or silane 2 (11-Aminoundecyltriethoxysilane) isopropyl alcohol solution (95% in water) at 37 ºC for 1 hour. Those discs dipped in 3-Aminopropyltriethoxysilane yielded a comparator material that is not of the present invention, while the discs dipped in 11-Aminoundecyltriethoxysilane yielded osteoinductive materials of the invention.

The titanium discs were then rinsed twice with isopropyl alcohol, ultra-pure water and ethanol and then dried at 135 °C for half an hour and stored in a vacuum desiccator.

### 4.2 Surface stability study

The shelf life of medical devices is a crucially important factor for the healthcare provider and the manufacturer. To study the stability of the coatings, the same surface modification strategy has been used to modify coverslips. The modified coverslips were stored in dry air, water for 1, 2 and 4 weeks for investigating the stability of the coating.

Two methods have been used to sterilize the silane modified coverslips for studying their effect on the stability of the coating. The first method is to sterilize the coverslips by 70% ethanol for 15 mins; the other is to autoclave the coverslips at 121ºC for 15 mins.

### 4.3 Materials characterization

### 4.3.1 Scanning Electronic Microscopy (SEM)

SEM was performed using a Leo/Zeiss 1550 Field Emission Scanning Electron Microscope. Briefly, one titanium disc from each group (control, comparator, or material of the invention) was mounted onto an aluminium stub using double sided adhesive carbon tape and sputter coated with chromium using an EMITECH K575X coater. The surface micro-structures and profiles were observed using Field Emission Scanning Electron Microscopy (FE-SEM) (LEO 1550, Cambridge, UK). The coated sample was placed in the vacuum chamber of the SEM and viewed at a voltage of 5 kV.

### 4.3.2 X-ray element analysis

X-ray analysis was performed using Oxford Instruments INCA software version 4.15. The system was calibrated using a MAC (Micro Analysis Consultants Ltd) Standard, number 5383, using the unmodified (control) titanium sample. 5 areas of the sample surface were chosen at random, at magnification x1000, and the area scanned for 5 mins using the ANALYZER function.

### 4.3.3 Contact angle

Dynamic contact angles of the coverslips samples with or without modification in deionised purified water were analysed using the Wilhelmy contact angle test. Briefly, the contact angles for all the samples were determined using a Dynamic Contact Angle Tensiometer (CDCA 100, Camtel Ltd., Royston, Herts, UK) at 22 ± 0.5 ºC. Each sample was immersed into, and retracted from the wetting solution (deionised pure water) at a rate of 0.060 mm/s. The wetting force at the solid/liquid/vapour interface was recorded via the electrobalance as a function of both time and immersion depth, and was converted into advancing contact angles. The values reported for dynamic advancing angles of are mean and standard deviations of four measurements.

### 5 Results

### 5.1 Surface modification of Ti discs

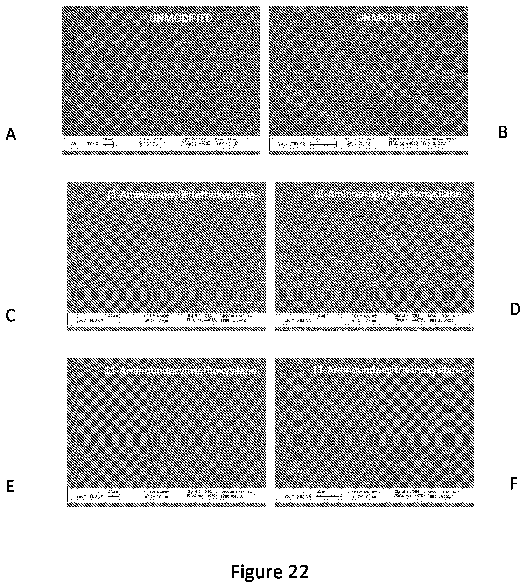

Scanning electron microscopy images illustrating the surfaces of the control ("unmodified"), comparator materials ("(3-Aminopropyl)triethoxysilane"), and materials of the invention ("11-Aminoundecyltriethoxysilane") are shown in Figure 22.

The results of surface element analysis results of titanium discs before and after amine silane modification are set out in Table 2.

**Table 2: Elements composition of Ti discs surface with/without modification**

| Element (atomic %) | Ti discs | 3-Aminopropyltriethoxysilane | 11-Aminoundecyltriethoxysilane |
|---|---|---|---|
| C | 0.00 ± 0.26 | 11.96 ± 0.62 | 15.59 ± 2.00 |
| Si | 2.14 ± 0.26 | 1.56 ± 0.03 | 1.59 ± 0.06 |
| Ti | 97.86 ± 0.26 | 86.36 ± 0.79 | 82.78 ± 2.00 |

The SEM images show that after silane modification, the surface of titanium discs became smoother, especially after modification by silane with long methylene chains (i.e. in the osteoinductive materials of the invention modified with 11-Aminoundecyltriethoxysilane). Element analysis result shows carbon increases and titanium decreases after silane modification. These results indicated that the surfaces of treated titanium discs have been successfully modified with amine terminated silanes.

### 5.2 Surface modification stability

To study the stability of the silane modification, the surface contact angles were measured with glass coverslips which have been untreated, or modified with the same strategy (treatment with either 3-Aminopropyltriethoxysilane to produce a comparator material, or 11-Aminoundecyltriethoxysilane to produce an osteoinductive material of the invention).

**Table 3: Contact angles results of coverslips under different sterilization and storage conditions**

| **Contact angles (°)** | | **Unmodified Coverslips** | **3-Aminopropyltriethoxysilane** | **11-Aminoundecyltriethoxysilane** |
|---|---|---|---|---|
| | | 49.90 ± 3.66 | 75.45 ± 1.63 | 91.39 ± 1.41 |
| Autoclave (121 ºC) | | 47.37 ± 5.79 | 71.51 ± 3.11 | 89.55 ± 4.40 |
| Ethanol (70%) | | 49.22 ± 4.31 | 73.18 ± 4.95 | 88.73 ± 3.31 |
| Water | 1 week | ------- | 67.19 ± 1.76 | 82.02 ± 2.69 |
| | 2 week | ------- | 67.34 ± 3.00 | 85.05 ± 3.45 |
| | 4 week | ------- | 66.94 ± 2.13 | 81.54 ± 7.74 |
| Air | 1 week | ------- | 77.86 ± 8.76 | 90.21 ± 5.79 |
| | 2 week | ------- | 75.49 ± 2.95 | 92.33 ± 5.15 |
| | 4 week | ------- | 72.61 ± 4.83 | 89.36 ± 7.33 |

The results set out in Table 3 shows that sterilisation utilising an autoclave (standard 121°C sterilisation) and 70% ethanol sterilisation, did not affect the overall hydrophobic/hydrophilic properties of the material. Slight changes in contact angle were within acceptable limits and did not affect the overall properties of the surface. The result indicate that osteoinductive materials of the invention, as exemplified by the silane modified titanium discs, can be sterilized by autoclave at 121 ºC or with 70% ethanol with no detrimental effect to the properties of the modified surfaces.

The result in Table 3 also shows that the silane modifications of coverslips are stable in water or dry air at least for 4 weeks.

### 6 Analysis of the osteoinductive properties of the materials

As with previous cell testing detailed in Study I above, modified and unmodified titanium discs were cultured in contact with commercially available well characterised human mesenchymal stem cells (Lonza), in basal medium. The ability of the materials to induce osteogenic differentiation was characterised qualitatively using markers for collagen I (major component of extracellular matrix) and osteocalcin (marker of osteogenic differentiation).

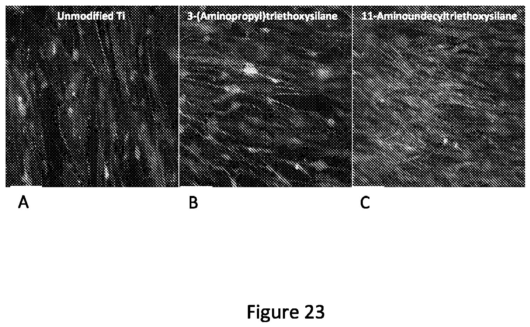

Photomicrographs of cells grown on control materials ("unmodified"), comparator materials ("3-(Aminopropyl)triethoxysilane"), and osteoinductive materials of the invention ("11-Aminoundecyltriethoxysilane") are shown in Figure 23. The images are of MSCs cultured in contact with the various materials utilising titanium substrates for 28 days and stained for osteocalcin (red), F-actin (marker of cellular cytoskeleton, green) and cell nuclei (blue). Cells cultured in contact with the titanium discs treated with 11-Aminoundecyltriethoxysilane to produce materials of the invention demonstrated a greater level of osteocalcin expression compared to un-modified (control) and comparator substrates modified with 3-(Aminopropyl)triethoxysilane).

Data retrieved from the study proved that cells cultured in contact with 11-Aminoundecyltriethoxysilane in materials of the invention showed a greater level of osteogenic activity compared to other amine modified and unmodified titanium substrates. This data is in line with previously provided data regarding the optimal osteogenic properties of amine modified glass and PLGA.

## Claims

1. An osteoinductive material comprising:
• a substrate; and
• a layer of a hydrocarbyl group comprising 10 to 12 carbon atoms, the hydrocarbyl group having one end attached to the substrate and a presenting amine (-NH2) group at the unattached end, wherein the substrate is in the form of a cell culture vessel or a medical implant selected from the group consisting of: an orthopaedic implant; a dental implant; and a part of an orthopaedic or dental implant.

2. An osteoinductive material according to claim 1, wherein the substrate comprises one or more constituents selected from the group consisting of: glass, metals; polymers; ceramics; plastics; and hydroxyapatite.

3. An osteoinductive material according to claim 1 or claim 2, wherein the hydrocarbyl group is an 11 carbon alkyl chain.

4. An osteoinductive material according to any preceding claim, wherein the hydrocarbon is attached to the substrate via a Si-O-Si bond.

5. An osteoinductive material according to any preceding claim, wherein the hydrocarbyl group is the only hydrocarbon present on a hydrocarbyl group-coated surface of the osteoinductive material.

6. An osteoinductive material according to any preceding claim, comprising multiple layers of the hydrocarbyl group and presenting amine.

7. An osteoinductive material according to claim 6, wherein the multiple layers are distributed at different depths over the surface of the material and wherein the multiple layers provide nanometre-scale features on the surface of the osteoinductive material.

8. An osteoinductive material according to claim 7, wherein the substrate comprises a polymer and the maximum feature height is 100 nm.

9. An osteoinductive material according to claim 7, wherein the substrate comprises glass and the maximum feature height is 140 nm.

10. An osteoinductive material according to any preceding claim, wherein the water contact angle at the surface of the material is less than 100°.

11. A method of producing an osteoinductive material, the method comprising contacting a surface of a substrate with a solution containing a compound comprising a hydrocarbyl group comprising 10 to 12 carbon atoms and a terminal amine group, such that a coating of the hydrocarbyl group is formed on at least a portion of a surface of the substrate.

12. A method according to any of claim 11, wherein the substrate comprises one or more constituents selected from the group consisting of: glass, metals; polymers; ceramics; plastics; and hydroxyapatite.

13. A method according to claims 11 or 12, wherein the hydrocarbyl group is 11 carbons long.

14. A method according to claim 13, wherein the hydrocarbyl group is provided in the form of 11-aminoundecyltriethoxysilane.

15. A method according to any of claims 11-14, wherein the compound comprising the hydrocarbyl group is provided at a concentration of 0.1 M.

16. A method according to any of claims 11 to 15, wherein the surface of the substrate is contacted with the solution by immersion of the substrate within the solution, or by spraying the solution onto the surface of the substrate.

17. A method according to any of claims 11 to 16, further comprising washing the coated substrate in alcohol and/or water.

18. A method according to any of claims 11 to 17, further comprising drying the surface of the substrate.

19. A method according to any of claims 11 to 18, wherein the substrate is in the form of a medical implant or part thereof, an orthopaedic implant or part thereof, a dental implant or part thereof, or a cell culture vessel.

20. A method according to any of claims 11 to 19, wherein the osteoinductive material is as defined in any of claims 1 to 10.

21. The use of an osteoinductive material according to any one of claims 1 to 10, or produced by a method according to any one of claims 11 to 20, for the production of bone or a bone precursor.

22. A method of producing bone or a bone precursor ex vivo, the method comprising:
• providing an osteoinductive material according to any of claims 1 to 10, or produced by a method according to any of claims 11 to 20;
• providing a population of cells with osteogenic potential; and
maintaining the cells in contact with the material until bone or a bone precursor is produced.

23. A method according to claim 22, wherein the cells with osteogenic potential are stem cells.

24. A method according to claim 23, wherein the stem cells are selected from the group consisting of: embryonic stem cells; cord blood stem cells; adult stem cells (including haematopoietic stem cells; mesenchymal stem cells; and dental pulp stem cells); and induced pluripotent stem cells.

25. A method according to any of claims 22 to 24, further comprising maintaining the cells in contact with the material until the bone precursor is remodelled into bone or a bone-like substance.

26. A method according to any of claims 22 to 25, wherein the cells are maintained in serum-free cell culture medium.

27. A method according to any of claims 22 to 26, wherein the cells are maintained in cell culture medium without osteogenic growth factors.

28. A method according to claim 27, wherein the cells are maintained in cell culture medium substantially free of exogenous growth factors.

## Patentansprüche

1. Osteoinduktives Material, umfassend:
• ein Substrat; und
• eine Schicht einer Hydrocarbyl-Gruppe, umfassend 10 bis 12 Kohlenstoffatome,
wobei die Hydrocarbyl-Gruppe ein Ende, das an dem Substrat befestigt ist, und eine vorhandene Amin(-NH2)-Gruppe an dem freien Ende aufweist, wobei das Substrat in der Form eines Zellkulturgefäßes oder eines medizinischen Implantats ist, ausgewählt aus der Gruppe bestehend aus: einem orthopädischen Implantat; einem Zahnimplantat; und einem Teil von einem orthopädischen Implantat oder einem Zahnimplantat.

2. Osteoinduktives Material nach Anspruch 1, wobei das Substrat ein oder mehrere Bestandteile umfasst, ausgewählt aus der Gruppe bestehend aus: Glas, Metallen; Polymeren; Keramiken; Kunststoffen; und Hydroxylapatit.

3. Osteoinduktives Material nach Anspruch 1 oder Anspruch 2, wobei die Hydrocarbyl-Gruppe eine Alkylkette mit 11 Kohlenstoffatomen ist.

4. Osteoinduktives Material nach einem der vorhergehenden Ansprüche, wobei der Kohlenwasserstoff an dem Substrat mittels einer Si-O-Si-Bindung befestigt ist.

5. Osteoinduktives Material nach einem der vorhergehenden Ansprüche, wobei die Hydrocarbyl-Gruppe der einzige Kohlenwasserstoff ist, der auf einer mit einer Hydrocarbyl-Gruppe beschichteten Oberfläche des osteoinduktiven Materials vorhanden ist.

6. Osteoinduktives Material nach einem der vorhergehenden Ansprüche, umfassend mehrere Schichten der Hydrocarbyl-Gruppe und des vorhandenen Amins.

7. Osteoinduktives Material nach Anspruch 6, wobei die mehreren Schichten in unterschichtlichen Tiefen über die Oberfläche des Materials verteilt sind und wobei die mehreren Schichten nanoskalige Merkmale auf der Oberfläche des osteoinduktiven Materials bereitstellen.

8. Osteoinduktives Material nach Anspruch 7, wobei das Substrat ein Polymer umfasst und die maximale Merkmalshöhe 100 nm beträgt.

9. Osteoinduktives Material nach Anspruch 7, wobei das Substrat Glas umfasst und die maximale Merkmalshöhe 140 nm beträgt.

10. Osteoinduktives Material nach einem der vorhergehenden Ansprüche, wobei der Wasserkontaktwinkel auf dem Material weniger als 100° beträgt.

11. Verfahren zum Herstellen eines osteoinduktiven Materials, wobei das Verfahren das Inkontaktbringen einer Oberfläche eines Substrats mit einer Lösung umfasst, die eine Verbindung enthält, die eine Hydrocarbyl-Gruppe umfasst, die 10 bis 12 Kohlenstoffatome und eine terminale Amin-Gruppe umfasst, sodass eine Beschichtung der Hydrocarbyl-Gruppe zumindest auf einem Abschnitt einer Oberfläche des Substrats gebildet wird.

12. Verfahren nach Anspruch 11, wobei das Substrat ein oder mehrere Bestandteile umfasst, ausgewählt aus der Gruppe bestehend aus: Glas, Metallen; Polymeren; Keramiken; Kunststoffen; und Hydroxylapatit.

13. Verfahren nach Anspruch 11 oder 12, wobei die Hydrocarbyl-Gruppe 11 Kohlenstoffatome aufweist.

14. Verfahren nach Anspruch 13, wobei die Hydrocarbyl-Gruppe in der Form eines 11-Aminoundecyltriethoxysilan bereitgestellt wird.

15. Verfahren nach einem der Ansprüche 11 - 14, wobei die Verbindung, die Hydrocarbyl-Gruppe umfasst, in einer Konzentration von 0,1 M bereitgestellt wird.

16. Verfahren nach einem der Ansprüche 11 - 15, wobei die Oberfläche des Substrats mit der Lösung durch Eintauchen des Substrats in die Lösung oder durch Aufsprühen der Lösung auf die Oberfläche des Substrats in Kontakt gebracht wird.

17. Verfahren nach einem der Ansprüche 11 bis 16, ferner umfassend das Waschen des beschichteten Substrats in Alkohol und/oder Wasser.

18. Verfahren nach einem der Ansprüche 11 bis 17, ferner umfassend das Trocknen der Oberfläche des Substrats.

19. Verfahren nach einem der Ansprüche 11 bis 18, wobei das Substrat in der Form eines medizinischen Implantas oder eines Teils davon, eines orthopädischen Implantats oder eines Teils davon, eines Zahnimplantats oder eines Teils davon oder eines Zellkulturgefäßes ist.

20. Verfahren nach einem der Ansprüche 11 bis 19, wobei das osteoinduktive Material wie nach einem der Ansprüche 1 bis 10 definiert ist.

21. Verwendung eines osteoinduktiven Materials nach einem der Ansprüche 1 bis 10 oder hergestellt durch ein Verfahren nach einem der Ansprüche 11 bis 20 zur Herstellung eines Knochens oder Knochenvorläuferzellen.

22. Verfahren zum Herstellen von Knochen oder Knochenvorläuferzellen ex vivo, wobei das Verfahren Folgendes umfasst:
• Bereitstellen eines osteoinduktiven Materials nach einem der Ansprüche 1 bis 10 oder hergestellt durch ein Verfahren nach einem der Ansprüche 11 bis 20;
• Bereitstellen einer Zellpopulation mit osteogenem Potential; und
Inkontakthalten der Zellen mit dem Material bis ein Knochen oder Knochenvorläuferzellen hergestellt wird/werden.

23. Verfahren nach Anspruch 22, wobei die Zellen mit osteogenem Potential Stammzellen sind.

24. Verfahren nach Anspruch 23, wobei die Stammzellen aus der Gruppe bestehend aus: embryonalen Stammzellen; Stammzellen aus Nabelschnurblut; adulten Stammzellen (einschließlich hämatopoetischen Stammzellen; Mesenchym-Stammzellen; und Zahnpulpstammzellen); und induzierten pluripotenten Stammzellen ausgewählt werden.

25. Verfahren nach einem der Ansprüche 22 bis 24, ferner umfassend das Inkontakthalten der Zellen mit dem Material bis Knochenvorläuferzellen in einen Knochen oder einer knochenähnlichen Substanz umgeformt werden.

26. Verfahren nach einem der Ansprüche 22 bis 25, wobei die Zellen in serumfreien Zellkulturmedium gehalten werden.

27. Verfahren nach einem der Ansprüche 22 bis 26, wobei die Zellen in einem Zellkulturmedium ohne osteogenen Wachstumsfaktoren gehalten werden.

28. Verfahren nach einem der Ansprüche 27, wobei die Zellen in einem Zellkulturmedium gehalten werden, das im Wesentlichen frei von exogenen Wachstumsfaktoren ist.

## Revendications

1. Matériau ostéo-inducteur comprenant :
• un substrat ; et
• une couche d'un groupe hydrocarbyle comprenant 10 à 12 atomes de carbone, le groupe hydrocarbyle ayant une extrémité attachée au substrat et un groupe amine (-NH2) présenté à l'extrémité non attachée, ledit substrat étant sous la forme d'un récipient de culture cellulaire ou d'un implant médical choisi dans le groupe constitué par : un implant orthopédique, un implant dentaire et une partie d'implant orthopédique ou dentaire.

2. Matériau ostéo-inducteur selon la revendication 1, ledit substrat comprenant un ou plusieurs constituants choisis dans le groupe constitué par : le verre, les métaux ; les polymères, les céramiques ; les plastiques ; et l'hydroxyapatite.

3. Matériau ostéo-inducteur selon la revendication 1 ou 2, ledit groupe hydrocarbyle étant une chaîne alkyle de 11 atomes de carbone.

4. Matériau ostéo-inducteur selon l'une quelconque des revendications précédentes, le groupe hydrocarboné étant attaché au substrat par une liaison Si-O-Si.

5. Matériau ostéo-inducteur selon l'une quelconque des revendications précédentes, ledit groupe hydrocarbyle étant le seul groupe hydrocarboné présent sur la surface enduite de groupe hydrocarbyle du matériau ostéo-inducteur.

6. Matériau ostéo-inducteur selon l'une quelconque des revendications précédentes, comprenant de multiples couches du groupe hydrocarbyle et du groupe amine présenté.

7. Matériau ostéo-inducteur selon la revendication 6, lesdites multiples couches étant réparties à différentes profondeurs sur la surface du matériau et lesdites multiples couches procurant des caractéristiques à l'échelle nanométrique sur la surface du matériau ostéo-inducteur.

8. Matériau ostéo-inducteur selon la revendication 7, ledit substrat comprenant un polymère et la hauteur caractéristique maximale étant égale à 100 nm.

9. Matériau ostéo-inducteur selon la revendication 7, ledit substrat comprenant du verre et la hauteur caractéristique maximale étant égale à 140 nm.

10. Matériau ostéo-inducteur selon l'une quelconque des revendications précédentes, l'angle de contact de l'eau au niveau de la surface du matériau étant inférieur à 100°.

11. Procédé de production d'un matériau ostéo-inducteur, le procédé comprenant la mise en contact d'une surface d'un substrat avec une solution contenant un composé comprenant un groupe hydrocarbyle comprenant 10 à 12 atomes de carbone et un groupe amine terminal, de sorte qu'un revêtement du groupe hydrocarbyle soit formé sur au moins une partie d'une surface du substrat.

12. Procédé selon la revendication 11, ledit substrat comprenant un ou plusieurs constituants choisis dans le groupe constitué par : le verre, les métaux ; les polymères ; les céramiques ; les plastiques ; et l'hydroxyapatite.

13. Procédé selon la revendication 11 ou 12, ledit groupe hydrocarbyle faisant 11 atomes de carbone de long.

14. Procédé selon la revendication 13, le groupe hydrocarbyle étant fourni sous la forme du 11-aminoundécyltriéthoxysilane.

15. Procédé selon l'une quelconque des revendications 11 à 14, ledit composé comprenant le groupe hydrocarbyle étant fourni à une concentration de 0,1 M.

16. Procédé selon l'une quelconque des revendications 11 à 15, ladite surface du substrat étant mise en contact avec la solution par immersion du substrat au sein de la solution ou par pulvérisation de la solution sur la surface du substrat.

17. Procédé selon l'une quelconque des revendications 11 à 16, comprenant en outre le lavage du substrat enduit dans de l'alcool et/ou de l'eau.

18. Procédé selon l'une quelconque des revendications 11 à 17, comprenant en outre le séchage de la surface du substrat.

19. Procédé selon l'une quelconque des revendications 11 à 18, ledit substrat étant sous la forme d'un implant médical ou d'une partie de celui-ci, d'un implant orthopédique ou d'une partie de celui-ci, d'un implant dentaire ou d'une partie de celui-ci, ou d'un récipient de culture cellulaire.

20. Procédé selon l'une quelconque des revendications 11 à 19, ledit matériau ostéo-inducteur étant tel que défini dans l'une quelconque des revendications 1 à 10.

21. Utilisation d'un matériau ostéo-inducteur selon l'une quelconque des revendications 1 à 10 ou produit par un procédé selon l'une quelconque des revendications 11 à 20, pour la production d'un os ou d'un précurseur d'os.

22. Procédé de production d'un os ou d'un précurseur d'os ex vivo, le procédé comprenant :
• la fourniture d'un matériau ostéo-inducteur selon l'une quelconque des revendications 1 à 10 ou produit par un procédé selon l'une quelconque des revendications 11 à 20 ;
• la fourniture d'une population de cellules dotées d'un potentiel ostéogénique ; et
le maintien des cellules en contact avec le matériau jusqu'à la production d'un os ou d'un précurseur d'os.

23. Procédé selon la revendication 22, lesdits cellules dotées d'un potentiel ostéogénique étant des cellules souches.

24. Procédé selon la revendication 23, lesdites cellules souches étant choisies dans le groupe constitué par : les cellules souches embryonnaires, les cellules souches de sang de cordon ombilical, les cellules souches adultes (dont les cellules souches hématopoïétiques, les cellules souches mésenchymateuses et les cellules souches de pulpe dentaire) et les cellules souches pluripotentes induites.

25. Procédé selon l'une quelconque des revendications 22 à 24, comprenant en outre le maintien des cellules en contact avec le matériau jusqu'au remodelage du précurseur d'os en un os ou en une substance de type os.

26. Procédé selon l'une quelconque des revendications 22 à 25, lesdites cellules étant maintenues dans un milieu de culture cellulaire exempt de sérum.

27. Procédé selon l'une quelconque des revendications 22 à 26, lesdites cellules étant maintenues dans un milieu de culture cellulaire sans facteurs de croissance ostéogéniques.

28. Procédé selon la revendication 27, lesdites cellules étant maintenues dans un milieu de culture cellulaire pratiquement exempt de facteurs de croissance exogènes.
